# EUROPÄISCHE PATENTANMELDUNG

(11) **EP 1 500 660 A1**
(43) Veröffentlichungstag der Anmeldung: **26.01.2005**
(21) Anmeldenummer: 02807142.1
(22) Anmeldetag: 20.12.2002
(51) Int. Cl.: C07H 7/033

(54) **VERFAHREN ZUR HERSTELLUNG VON D-GLUCORONSÄURE**

(30) Priorität: 02.04.2002 RU 2002108340
(71) Anmelder: Obschestvo S Ogranichennoi Otvetstvennoctiyu "MACFERON", Moscow 123242 (RU)
(72) Erfinder: BELJANIN, Maksim Lvovich, Tomsk, 634061 (RU); GOLDBERG, Evgeniy Danilovich, Tomsk, 634034 (RU); DYGAI, Alexandr Michailovich, Tomsk, 634034 (RU); FILIMONOV, Viktor Dmitrievich, Tomsk, 634061 (RU); KHAZANOV, Veniamin Abramovich, Tomsk, 634034 (RU)
(74) Vertreter: Jeck, Anton, Dipl.-Ing.
(86) Internationale Anmeldenummer: PCT/RU2002/000542
(87) Internationale Veröffentlichungsnummer: WO 2003/082886

(57) **Zusammenfassung**

Die Erfindung bezieht sich auf ein Verfahren zur Gewinnung von D-Glukuronsäure. Die D-Glukuronsäure und ihre Ableitungen (Lakton, Salze, Amide usw.) sind biologisch hochaktive Verbindungen, die eine breite Verwendung in der Medizin und pharmazeutischen Chemie zur Synthese von modifizierten Arzneimitteln sowie als Nahrungsmittelzusätze, Hautpflegemittel usw. finden.

Die Hauptaufgabe der vorliegenden Erfindung ist die Schaffung eines umweltfreundlichen Verfahrens zur Gewinnung von D-Glukuronsäure, das breite funktionale Möglichkeiten besitzt.

Diese Aufgabe wird folgenderweise gelöst: bei dem Verfahren erfolgt die Gewinnung der D-Glukuronsäure durch Erwärmung der Salze der 1,2-O-Isopropyliden-D-Glukuronsäure in einer wasserhaltigen Lösung in Anwesenheit eines Säureagens, für das sulfonsaure Kationenaustauscherharze verwendet werden.

## Beschreibung

Die Erfindung bezieht sich auf ein Verfahren zur Gewinnung von D-Glukuronsäure. Die D-Glukuronsäure und ihre Ableitungen (Lakton, Salze, Amide usw.) sind biologisch hochaktive Verbindungen, die eine breite Verwendung in der Medizin und pharmazeutischen Chemie zur Synthese von modifizierten Arzneimitteln sowie als Nahrungsmittelzusätze, Hautpflegemittel usw. finden.

Bei den meisten Verfahren zur Gewinnung von D-Glukuronsäure wird von der 1,2-Isopropyliden-D-Glukose ausgegangen, bei deren Oxidation 1,2-Isopropyliden-D-Glukuronsäure oder häufig deren Salze (Na, K, Ca, Ba usw.) ausgeschieden wird bzw. werden. Für die Umwandlung dieser Verbindungen in Glukuronsäure ist es erforderlich, die Stadien der Neutralisierung der Salze und der Säurehydrolyse der 1,2-Isopropyliden-Gruppe durchzuführen, was sich am folgenden Schema darstellen lässt (am Beispiel von Natriumsalz): SIEHE ZEICHNUNG

Die Glukuronsäure zeichnet sich durch eine hohe chemische Labilität aus und ist zu vielen Umwandlungen unter milden Bedingungen fähig. Die für Monosacharide typischen tautomeren Umwandlungen in Lösungen (Mutarotation) im Fall der Glukuronsäure werden durch die Bildung von D-Glukofuranyron-6,3-Lakton (Glukuronsäure-Lakton) erschwert. Das Gleichgewichtsverhältnis der Komponenten bei Zimmertemperatur beträgt 60 % Glukuronsäure und 40 % Glukuronsäure-Lakton. Die Erhöhung der Temperatur und das Vorhandensein von Säurekatalysatoren beschleunigen die Erreichung dieses Gleichgewichts. Bei der Erwärmung der D-Glukuronsäure in Anwesenheit von starken Säuren läuft auch das Dekarboxylieren unter Bildung von Kohlenstoffdioxid, Furfural und anderen Produkten der Zersetzung leicht ab. In den sauren, neutralen und alkalischen Medien wandelt sich die Glukuronsäure in Epimere nach C-2 und weiterhin in die jeweiligen Ketosäuren und isomere GK-Aldehydsäuren um. Dieser Umstand erschwert in hohem Maße die Prozesse der Gewinnung von Glukuronsäure (GS) und führt dazu, dass bei allen bekannten Verfahren entweder die Gemische von GS oder die Salze von Glukuron (GK) gewonnen werden.

Es ist ein Verfahren zur Gewinnung von 1,2-Isopropyliden-D-Natriumglukuronat bekannt (UdSSR Nr. 883054 M.KI.³ C07H7/02), das als Zwischenprodukt bei der Synthese der D-Glukuronsäure, deren Laktons (Glukuron) und deren Derivate eingesetzt wird, die durch eine katalytische Oxydation der 1,2-Isopropyliden-D-Glukose mit Luftsauerstoff im schwachbasischen Medium bei erhöhter Temperatur und unter Umrühren gewonnen werden. Bei der Oxydation wird als Katalysator ein Palladiumkatalysator verwendet, der 1,8 % Pd in Bezug auf Kohle hat, gefördert durch Kaliumsalze (0,8 %) und Natriumsalze (0,2%) bei einem Verhältnis der zu oxidierenden Verbindung und des Katalysators von 1:0,45-0,91 bei einer Temperatur von 65-70° C, einem pH-Wert von 7,5-8,0 und einer Konzentration des Ausgangstoffs von 1-10 %.

Es ist jedoch unmöglich, mit diesem Verfahren D-Glukuronsäure und Glukuron-Lakton zu gewinnen.

Es ist ein weiteres Verfahren zur Gewinnung von D-Glukuronsäure bekannt (Mehltretter C.L., Alexander B.H., Mellies R.L. et al. "A Practical Synthesis of D-Glucuronic Acid through the Catalytic Oxidation of 1,2-Isopropylidene-D-glucose", J. Am. Chem. Soc., 1951, V. 73, Nr. 6, Seiten 2424-2427). Bei diesem Verfahren wird die Wasserlösung von Ca-Isopropyliden-Glukuronsäure bei einer Temperatur von 90-100° C mit einer äquimolekularen Menge des Dihydrats der Oxalsäure im Laufe von 1,75 Stunden erwärmt, und dann wird das unlösliche und ausgefallene Kalziumoxalat gefiltert. Nach dem Entfernen des Wassers und nach dem Bearbeiten der Reaktionsmasse mit Äthanol wird ein Gemisch gewonnen, das 60% Glukuronsäure und 40 % Glukuron-Lakton (GK-Lakton) enthält.

Dieses Verfahren hat nur begrenzte Funktionsmöglichkeiten, denn es ist nur für Kalziumsalze (oder Bariumsalze) der 1,2-O-Isopropyliden-D-Glukuronsäure anwendbar, deren Neutralisierung und Hydrolyse zu unlöslichen Kalzium- oder Bariumoxalaten führt, die leicht von der GK-Lösung und dem GK-Lakton durch Filtern abzutrennen sind. Die anderen Salze der 1,2-O-Isopropyliden-D-Glukuronsäure (z. B. Natrium oder Kalium) liefern lösliche Oxalate, die vom Zielprodukt durch die bekannten Verfahren nicht abzutrennen sind.

Die Erfindung, die dem technischen Wesen und der zu lösenden Aufgabe der vorliegenden Erfindung am nächsten kommt, ist ein Verfahren zur Gewinnung von D-Glukuronsäure, die in Form eines Na-Salzes ausgeschieden wird (Feldmann D.P., Woytenko A.D., Schimaskaya M.B. usw., "Verfahren zur Gewinnung von D-Natriumglukuronat", Chemie-Pharm. Magazin, 1984, Nr. 11, Seiten 1356-1360). Bei diesem Verfahren wird die Azetonierung der D-Glukose in Anwesenheit von Schwefelsäure unter Bildung eines Gemischs aus zwei Hauptprodukten, nämlich 1,2-0-isopropyliden-D-Glukuronsäure und 1,2;5,6-di-O-Isopropyliden-D-Glukuronsäure durchgeführt, wobei die letzte Verbindung durch eine Säurehydrolyse in 1,2-0-Isopropyliden-D-Glukose umgewandelt und unter deren katalytischer Oxydation 1,2-O-Isopropyliden-D-Natriumglukuronat gewonnen wird. Durch die Hydrolyse der genannten Verbindung unter Erwärmen auf eine Temperatur von 100°C im Laufe von 2 Stunden und unter der Wirkung einer 1%igen Lösung der Chlorwasserstoffsäure und einer weiteren Neutralisierung mittels Natriumhydroxid und Eindampfen der Lösung bis zum trockenen Zustand wird ein Gemisch aus D-Natriumglukuronat und Natriumchlorid gewonnen. Die Ausfällung des D-Natriumglukuronats aus dem Gemisch wird durch eine mehrfache Extraktion mittels heißem Dimethylsulfoxid und ein späteres Zufügen von Azeton in den Extrakt sowie durch Filtern des ausfallenden D-Natriumglukuronats durchgeführt.

Die funktionellen Möglichkeiten des bekannten Verfahrens sind auf die Verwendung der 1,2-O-Isopropyliden-D-Glukuronsäure bei Metallen begrenzt, die in Dimethylsulfoxid unlösliche Chloride, z. B. NaCl, ergeben. Wenn die sich bildenden Chloride in Dimethylsulfoxid löslich sind, dann ist die Reinigung des D-Glukuronsäure-Salzes mittels der Extraktion unter Anwendung von heißem Dimethylsulfoxid nicht möglich.

Der Nachteil des bekannten Verfahrens ist auch, dass während der Neutralisierung und Hydrolyse der Na-Isopropyliden-Glukuronsäure mittels der Salzsäure als Ergebnis der Labilität der D-Glukuronsäure Nebenumwandlungen auftreten, die die Ausbeute reduzieren und die Reinigungsprozesse der Zielprodukte komplizierter machen. Außerdem führt die Reinigung des Na-Salzes der D-Glukuronsäure zu aufwendigen und umweltschädlichen Abläufen der Extraktion mittels Dimethylsulfoxid und ferner zu einem Absetzen des Produkts mit Azeton.

Die Hauptaufgabe, auf deren Lösung die vorliegende Erfindung gerichtet ist, ist das Schaffen eines umweltfreundlichen Verfahrens zur Gewinnung von D-Glukuronsäure, das breite funktionelle Möglichkeiten besitzt.

Diese Aufgabe wird dadurch gelöst, dass beim Verfahren zur Gewinnung von D-Glukuronsäure in einer wasserhaltigen Lösung in Anwesenheit eines Sauerstoffagens als Sauerstoffagens sulfonsaure Kationenaustauscherharze verwendet werden.

Optimal ist die Verwendung von sulfonsauren Kationenaustauscherharzen, die eine Austauschkapazität von nicht weniger als 4mg äqu/g in einer Menge von nicht weniger als 0,5 g auf 0,001 Mol der Salze der 1,2-O-Isopropyliden-D-Glukuronsäure aufweisen.

Es ist vorteilhaft, die Erwärmung der Salze bei Temperaturen von 40-95° C durchzuführen. Eine niedrigere Erwärmungstemperatur verlängert den Prozess der Hydrolyse bedeutend, während eine viel höhere Temperatur zu einer Reduzierung der Ausbeute der D-Glukuronsäure wegen der Labilität dieser Säure führt.

Es ist zweckmäßig, als wasserhaltige Lösung eine Wasserlösung aus Äthanol mit einer Konzentration von nicht mehr als 50 % wegen der schlechten Lösbarkeit der Salze der Isopropyliden-Glukuronsäure in Äthanol zu verwenden. Optimal ist, als Salze der 1,2-O-Isopropyliden-D-Glukuronsäure Alkali-, Erdalkali- und Ammoniumsalze zu verwenden, deren Kationen fähig sind, sich mit Kationenaustauscherharzen zu verbinden, z.B. Li, Na, K, Rb, Cs, Ca, Ba, Mg, Al, NH₄ usw.

Derzeit wurde von den Erfindern aus Informationsquellen kein Verfahren zur Gewinnung von D-Glukuronsäure im Umfang der vorliegenden Erfindung ermittelt.

Das Verfahren gemäß der Erfindung ist umweltfreundlich und sichert die Gewinnung eines reineren Produkts. Das Fehlen von homogenen Säurekatalysatoren (HCl, Oxalsäure und weitere Säuren) in der Lösung beugt Prozessen von Nebenumwandlungen der D-Glukuronsäure vor, die die Zielprodukte verschmutzen und eine Reinigung mittels aufwändiger und umweltschädigender Technologien erfordern. Die sulfonsauren Kationenaustauscherharze, die als Säureagens verwendet werden, lassen sich leicht von der Reaktionsmasse durch ein einfaches Filtern abtrennen und leicht mit bekannten Verfahren regenerieren.

Umfangreiche funktionelle Möglichkeiten des Verfahrens gemäß der Erfindung werden dadurch sichergestellt, dass bei dem Verfahren beliebige Salze der 1,2-0-Isopropyliden-D-Glukuronsäure verwendet werden, deren Kationen fähig sind, sich mit Kationenaustauscherharzen zu verbinden (Alkaliharze, Erdalkali- oder Ammoniumsalze, z. B., Li, Na, K, Rb, Cs, Ca, Ba, Mg, Al, NH₄ usw.).

Die Möglichkeit der Verwirklichung des Verfahrens gemäß der Erfindung in einer wasserhaltigen Lösung (vorwiegend in einer alkoholisch-wässrigen) erleichtert das Abziehen der Lösungsmittel im Laufe der Ausscheidung des GK und des GK-Laktons, weil das Zufügen des niedrigsiedenderen Alkohols die gesamte Siedetemperatur des Gemischs von Alkohol und Wasser senkt.

Die Technologie der Gewinnung der D-Glukuronsäure nach dem Verfahren gemäß der Erfindung verlangt keinen bedeutenden Aufwand, weil Rohstoffe und Reagens (Glukose, Wasser, Alkohol, Alkali, Sulfokationit), milde Bedingungen und eine Einfachheit der durchzuführenden technologischen Operationen (Umrühren, Filtern, Abtrennen des Lösungsmittels) leicht zu erlangen sind.

Die Verwendung von Kationenaustauscherharzen bei der Produktion von D-Glukuronsäure ist bekannt (Pat. Japan Nr. 15119, 1962, Chem. Abstr., 1963, V. 59, N 2136a; Patent Japan Nr. 1366, 1963, Chem. Abstr., 1964, V. 60, N 651 a; Imai Y., Hirasaka Y., "Zakugaku Zasshi", 1960, V. 80, Seiten 1139-1142). Diese Harze werden aber nur bei den Prozessen der Neutralisierung der Salze der D-Glukuronsäure verwendet. Gemäß der vorliegenden Erfindung sichern die sulfonsauren Kationen-austauscherharze neben der Neutralisierung der Salze der 1,2-O-Isopropyliden-D-Glukuronsäure beim Erwärmen die Abspaltung (Hydrolyse) der Schutz-Isopropyliden-Gruppe von den genannten Verbindungen.

Die letzte hydrolytische Funktion der sulfonsauren Kationenaustauscherharze ist im Gegensatz zu Salzen der 1,2-O-Isopropyliden-D-Glukuronsäure nicht eindeutig. Soweit aus den Informationsquellen bekannt ist, wurde sie früher zur Gewinnung der D-Glukuronsäure nicht verwendet.

Bei der Hydrolasierung und Hydrolyse der Na-Isopropyliden-Glukuronsäure mit Salzsäure bildet sich Natriumchlorid. Wenn sulfonsaure Kationenaustauscherharze verwendet werden, fehlt Natriumchlorid (oder andere Salze), d. h., diese Lösungen können nach der einfachen Abtrennung vom sulfonsauren Kationenaustauscherharz in verschiedenen Bereichen (Medizin, Lebensmittelindustrie) als unmittelbare Quelle der D-Glukuronsäure verwendet werden.

Die vorliegende Erfindung wird in folgender Weise realisiert:

Ein Gemisch aus einem Salz der 1,2-O-Isopropyliden-D-Glukuronsäure, z. B. Kalziumsalz der 1,2-O-Isopropyliden-D-Glukuronsäure des Pentahydrats, einer wasserhaltigen Lösung und einem sulfonsauren Kationenaustauscherharz, z. B. Amberlit-IR oder KU-2 (universeller Kationit), das vom Werk "Azot" in Kemerowo hergestellt wird, wird erwärmt.

Es werden sulfonsaure Kationenaustauscherharze verwendet, die eine Austauschkapazität von nicht weniger als 4 mg äqu/g in einer Menge von nicht weniger als 0,5g auf 0,001 Mol der Salze der 1,2-O-Isopropyliden-D-Glukuronsäure aufweisen.

Die Erwärmung erfolgt bei einer Temperatur von 40-95° C. Nach dem Abschluss der Reaktion wird das Kationenaustauscherharz gefiltert und mit Wasser gespült. Das Filtrat enthält ein Gemisch aus D-Glukuronsäure und D-Glukuronsäure-Lakton. Im genannten Temperaturbereich läuft die Reaktion innerhalb von 1-5 Stunden ohne Nebenprozesse der Zersetzung des GK ab, so dass eine Gesamtausbeute der Reaktionsprodukte (Summe GK und GK-Lakton) gewonnen wird. Das Verhältnis von GK und GK-Lakton im Produkt wird nur durch die Bedingungen des thermodynamischen Gleichgewichts zwischen ihnen (Temperatur und Dauer) bestimmt. Als wasserhaltige Lösung wird eine Wasserlösung aus Äthanol mit einer Konzentration von nicht mehr als 50 % verwendet. Als Salze der 1,2-O-Isopropyliden-D-Glukuronsäure werden Alkali-, Erdalkali- und Ammoniumsalze verwendet, deren Kationen fähig sind, sich mit Kationenaustauscherharzen zu verbinden, z. B. Li, Na, K, Rb, Cs, Ca, Ba, Mg, Al, NH₄ usw.

Das Abziehen der D-Glukuronsäure aus der gewonnenen Wasser- oder wasserorganischen Lösung ist mit vielen Verfahren möglich, z. B. durch die Neutralisierung von Lösungen aus Hydroxiden der Alkalimetalle und Gewinnung der jeweiligen Salze der D-Glukuronsäure, die ihrerseits in eine freie D-Glukuronsäure mit der Neutralisierung der Kationenaustauscherharze (Pat. Japan Nr. 15119, 1962, Chem. Abstr., 1963, V. 59, N 2136a; Patent Japan Nr. 1366, 1963, Chem. Abstr., 1964, V. 60, N 651a) oder mit der Bearbeitung von Trifluoressigsäure (UdSSR Nr.1089957, 1984) oder (im Fall von Kalzium- oder Bariumsalzen-GK) mit der Bearbeitung von Schwefelsäure (Mehltretter C.L., Alexander B.H., Mellies R.L. et al. "A Practical Synthesis of D-Glucuronic Acid through the Catalytic Oxidation of 1,2-Isopropylidene-D-glucose", J. Am. Chem. Soc., 1951, V. 73, Nr. 6, Seiten 2424 - 2427; Zervas L., Sessler P., "Ber. 1933", Bd. 66, Seiten 1326-1329; Ehrlich F., Rehorst K. "Ber. 1929", Bd. 62A, Seiten 628-634) umgewandelt werden kann.

Es ist jedoch eine direkte Verwendung der nach dieser Erfindung gewonnenen Wasser- und wasserorganischen Lösungen der GK und des GK-Laktons als biologisch aktive Verbindungen in den Fällen möglich, wenn es ohne unmittelbares Abziehen der D-Glukuronsäure und GK-Laktons erforderlich ist.

### Beispiel 1

Ein Gemisch wird im Laufe von 5 Stunden bei einer Temperatur von 40° C erwärmt, das aus 3,88 g (0,00641 Mol) Kalziumsalz der 1,2-O-Isopropyliden-D-Glukuronsäure, des Pentahydrats, 75ml Wasser und 14,8 g Kationenaustauscherharz KU-2 besteht. Das Kationenaustauscherharz wird gefiltert und mit 10ml Wasser gespült. Das Filtrat enthält ein Gemisch aus D-Glukuronsäure und D-Glukuronsäurelakton im ungefähr gleichen Verhältnis. Zu der gewonnenen Lösung wird Natriumhydroxid mit einem pH-Wert von 6.5-7 bei Zimmertemperatur tropfenweise unter Umrühren zugeführt. Aus der gewonnenen Lösung der Natriumsalz-D-Glukuronsäure wird Wasser bei einer Temperatur von nicht höher als 30° C entfernt. Der Rest wird aus dem Gemisch Äthanol-Wasser (6:5) kristallisiert. Es werden 1,23 g Natriumsalz-D-Glukuronsäure gewonnen. Die Verdünnung der Mutterlösung mit 96%igem Äthanol gibt noch 0,33 g des Produkts. Die gesamte Ausbeute beträgt also 1,56 g (52 %) des reinen Natriumsalzes der D-Glukuronsäure des Kristallhydrats mit einer Schmelztemperatur von 147-149° C.

Es werden 1,56 g Natriumsalz der D-Glukuronsäure des Kristallhydrats in 10 ml Wasser aufgelöst, dann wird es durch eine Säule durchgelassen, die mit 10 g Kationenaustauscherharz KU-2 gefüllt ist. Die Säule wird mit Wasser bis zu einer neutralen Reaktion des Auswaschwassers gespült. Aus der gewonnenen Lösung der D-Glukuronsäure wird Wasser bei einer Temperatur von nicht höher als 20° C entfernt. Es werden 1,26 g kristallisierte D-Glukuronsäure (50 % bei Umrechnung in Kalziumsalz der 1,2-O-Isopropyliden-D-Glukuronsäure des Kristallhydrats) gewonnen.

### Beispiel 2

Ein Gemisch wird im Laufe von 1 Stunde bei einer Temperatur von 95° C erwärmt, das aus 3,88 g (0,00641 Mol) Kalziumsalz der 1,2-O-Isopropyliden-D-Glukuronsäure des Pentahydrats, 75ml Wasser und 14,8 g Kationenaustauscherharz KU-2 besteht.

Die weiteren Operationen werden analog zum Beispiel 1 durchgeführt. Es werden 1,1 g kristallisierte D-Glukuronsäure (44 % bei Umrechnung in Kalziumsalz der 1,2-0-Isopropyliden-D-Glukuronsäure des Pentahydrats) gewonnen.

### Beispiel 3

Ein Gemisch wird im Laufe von 3 Stunden bei einer Temperatur von 75° C erwärmt, das aus 3,88 g (0,00641 Mol) Kalziumsalz der 1,2-O-Isopropyliden-D-Glukuronsäure des Pentahydrats, 40 ml Wasser, 40 ml 96%iges Äthanol und 14,8 g Kationenaustauscherharz KU-2 besteht. Das Kationenaustauscherharz wird gefiltert und mit 10ml Wasser gespült. Zu der gewonnenen Lösung wird bei Zimmertemperatur tropfenweise unter Umrühren eine Lösung aus Natriumhydroxid mit einem pH-Wert von 6.5-7 zugefügt. Aus der gewonnenen Lösung der Natriumsalz-D-Glukuronsäure werden Wasser und Äthylalkohol bei einer Temperatur von nicht höher als 30° C entfernt. Der Rest wird aus dem Gemisch Äthanol-Wasser (6:5) kristallisiert. Das gewonnene Natriumsalz der D-Glukuronsäure wird in 10 ml Wasser aufgelöst, dann wird es durch eine Säule durchgelassen, die mit 10 g Kationenaustauscherharz KU-2 gefüllt ist. Die Säule wird mit Wasser bis zur neutralen Reaktion des Auswaschwassers gespült. Aus der gewonnenen Lösung der D-Glukuronsäure wird Wasser bei einer Temperatur von nicht höher als 20° C entfernt. Es werden 1,23 g kristallisierte D-Glukuronsäure (49 % bei Umrechnung in Kalziumsalz der 1,2-O-Isopropyliden-D-Glukuronsäure des Pentahydrats) gewonnen.

### Beispiel 4

Ein Gemisch wird im Laufe von 3 Stunden bei einer Temperatur von 75° C erwärmt, das aus 3,28 g (0,0128 Mol) Natriumsalz der 1,2-O-Isopropyliden-D-Glukuronsäure, 75 ml Wasser und 14,8 g Kationenaustauscherharz KU-2 besteht. Das Kationenaustauscherharz wird gefiltert und mit 10 ml Wasser gespült. Das Filtrat enthält ein Gemisch aus D-Glukuronsäure und D-Glukuron (Lakton der D-Glukuronsäure) im ungefähr gleichen Verhältnis. Die gewonnene Lösung wird analog zum Beispiel 1 bearbeitet. Es werden 1,28 g kristallisierte D-Glukuronsäure (51,5 % bei Umrechnung in Natriumsalz der 1,2-O-Isopropyliden-D-Glukuronsäure) gewonnen.

### Beispiel 5

Ein Gemisch wird im Laufe von 3 Stunden bei einer Temperatur von 75° C erwärmt, das aus 3,88 g (0.00641) Kalziumsalz der 1,2-O-Isopropyliden-D-Glukuronsäure des Pentahydrats, 75 ml Wasser und 15 g Kationenaustauscherharz Amberlite IR-120 besteht. Das Kationenaustauscherharz wird gefiltert und mit 10 ml Wasser gespült.

Das Filtrat wird analog zum Beispiel 1 bearbeitet. Es werden 1,14 g kristallisierte D-Glukuronsäure (46 % bei Umrechnung in Kalziumsalz der 1,2-O-Isopropyliden-D-Glukuronsäure des Pentahydrats) gewonnen.

### Beispiel 6

Ein Gemisch wird im Laufe von 4 Stunden bei einer Temperatur von 75° C erwärmt, das aus 3,88 g (0,00641 Mol) Kalziumsalz der 1,2-O-Isopropyliden-D-Glukuronsäure des Pentahydrats, 75 ml Wasser und dem Kationenaustauscherharz KU-2 in einer Menge von nicht weniger als seine Volumenkapazität (2,56 g) besteht. Die weiteren Operationen werden analog zum Beispiel 1 ausgeführt. Es werden 1,03 g kristallisierte D-Glukuronsäure (41 % bei Umrechnung in Kalziumsalz der 1,2-0-Isopropyliden-D-Glukuronsäure des Pentahydrats) gewonnen.

Das Verfahren gemäß der Erfindung wurde erfolgreich im Versuchsmaßstab bei Verwendung von Chargen des Reagens in einer Menge von mehreren hundert Gramm erprobt. Das Verfahren zur Gewinnung von D-Glukuronsäure ist somit umweltfreundlich und verfügt über breite funktionelle Möglichkeiten.

### Beschreibung zur Zeichnung

| | |
|---|---|
| 1. Na - JPGK - | 1,2 - 0 - isopropylidene - D - glucuronic acic Natrium Salt |
| | 1,2 - 0 - isopropylidenglukuronsäure Natriumsalz |
| | |
| 2. JPGK | - 1,2 - 0 - isopropylidene - D - glucuronic acid |
| | - 1,2 - 0 - isopropylidenglukuronsäure |
| | |
| 3. Glukuronsäure | - D - Glucuronic acid |
| | D - Glukuronsäure |
| | |
| 4. D-Glukuron | - D - Glukurone |
| | D - Glukuron |

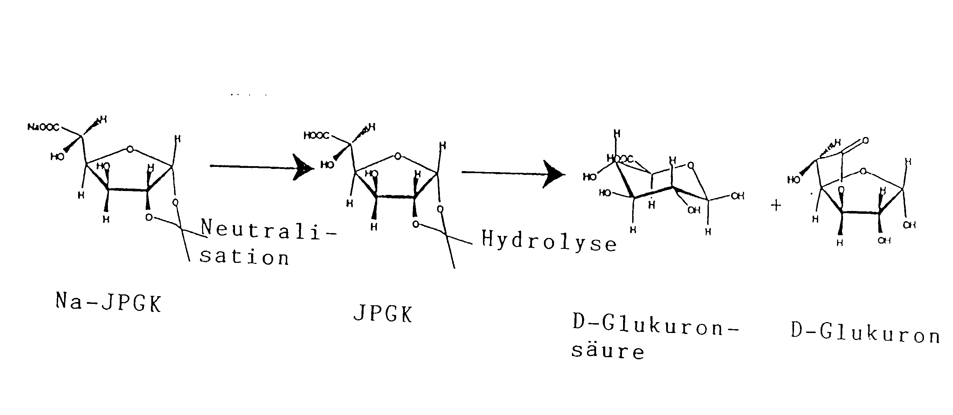

## Patentansprüche

1. Verfahren zur Gewinnung von D-Glukuronsäure durch Erwärmen der Salze der 1,2-O-Isopropyliden-D-Glukuronsäure in einer wasserhaltigen Lösung in Anwesenheit eines Säureagens,
**dadurch gekennzeichnet,**
**dass** als Säureagens sulfonsaure Kationenaustauscherharze eingesetzt werden.

2. Verfahren nach Anspruch 1,
**dadurch gekennzeichnet,**
**dass** als sulfonsaure Kationenaustauscherharze sulfonsaure Kationenaustauscherharze mit einer Volumenkapazität von nicht weniger als 4 mg äqu/g eingesetzt werden, und zwar in einer Menge von nicht weniger als 0,5 g auf 0,001 Mol Salze der 1,2-O-Isopropyliden-D-Glukuronsäure.

3. Verfahren nach Anspruch 1,
**dadurch gekennzeichnet,**
**dass** das Erwärmen der Salze der 1,2-O-Isopropyliden-D-Glukuronsäure bei einer Temperatur von 40-95° C durchgeführt wird.

4. Verfahren nach Anspruch 1,
**dadurch gekennzeichnet,**
**dass** als wasserhaltige Lösung eine Wasserlösung aus Äthanol mit einer Konzentration von nicht mehr als 50 % verwendet wird.

5. Verfahren nach Anspruch 1,
**dadurch gekennzeichnet,**
**dass** als Salze der 1,2-O-Isopropyliden-D-Glukuronsäure Salze der Alkali- und Erdalkalimetallgruppen oder Ammoniaksalze verwendet werden.
